# EUROPEAN PATENT APPLICATION

(11) **EP 1 210 957 A1**
(43) Date of publication of application: **05.06.2002**
(21) Application number: 01127342.2
(22) Date of filing: 20.11.2001
(51) Int. Cl.: A61M 16/00

(54) **Closed tracheal suction system**

(30) Priority: 24.11.2000 IT FO000032
(71) Applicant: D.E.A.S. S.R.L., 48014 Castelbolgnese (Prov. of Ravenna) (IT)
(72) Inventor: Scardovi, Domenico, 48014 Castelbolognese (Ravenna) (IT)
(74) Representative: Modiano, Guido, Dr.-Ing.

(57) **Abstract**

A closed tracheal suction system, comprising a Cobb connector, said connector consisting of a T-shaped connector coupled to an endotracheal tube (A) and to a tube (B) of a ventilation circuit, an end (C1) of said connector, which is the end that is aligned and opposite with respect to a coupling for the endotracheal tube (A), being shaped and sized so that after removing a closure cap (D) it can be inserted by pushing in a corresponding frustum-shaped seat (E1) of a connecting element (E) connected to a flexible protective sleeve (M) of a tracheal suction catheter (L).

## Description

Tracheal suction is a well-known procedure that, in intubated patients, allows to keep free from secretions the internal lumen of the endotracheal tube; the throwaway kit or assembly required to perform this operation by using a pneumatic or vacuum pump is normally termed tracheal suction system.

Various tracheal suction systems are currently commercially available and in some of them, known as "closed" systems, the catheter used to aspirate the secretions is protected by a flexible sleeve made of plastic material, in which one end is fixed in the connector for coupling to the ventilation circuit (Cobb connector) and the other end is fixed in the body of the valve for coupling the catheter to the suction pump.

The positive aspects of these closed systems include that of their possible reuse without the risk of bacterial contaminations of the respiratory pathways of the intubated patient, since the tracheal suction catheter passes directly from the flexible protection sleeve to the endotracheal tube and vice versa and is at all times isolated from the outside environment.

However, although they are widely used, current closed tracheal suction systems are all penalized by the fact that they are stably integrated, by means of the Cobb connector, with the ventilation circuit of the endotracheal tube.

In fact any removal of the closed tracheal suction system from the ventilation circuit of the already-intubated patient not only requires the availability of a new Cobb connector but also entails considerable difficulties and most of all entails gas leakages.

This is true also for the opposite case; accordingly, in order to avoid the mentioned severe drawbacks, whether to adopt the closed tracheal suction system cannot be decided according to the requirements but must be decided right from the moment when the patient is intubated.

In view of the above described situation, the aim of the present invention is to make it easier and quicker, at any time and even in intubated patients, to perform both connection and removal of the closed tracheal suction system with respect to the Cobb connector and therefore with respect to the ventilation circuit.

This is to be achieved without having to replace any component and without interrupting the ventilation of the intubated patient.

Since in current closed tracheal suction systems it is possible to wash the end of the catheter in an appropriately provided chamber which is connected to a water supply duct and is located in the Cobb connector or in an element that is stably connected thereto, one of the objects of the present invention is accordingly also to prevent, during the washing of the catheter and of the collected secretions, both the escape of gas and the passage of water in the endotracheal tube.

The aim of the invention is to provide the possibility of easily separating the closed tracheal suction system from the ventilation circuit.

Another object is to prevent, during this separation, the exposure of the catheter to the outside environment, since mere concern for possible bacterial contaminations of the respiratory pathways of the intubated patient would prevent its subsequent reuse.

This aim and these and other objects which will become better apparent hereinafter are achieved by providing a closed tracheal suction system as described hereinafter with the aid of the following drawings, given only by way of non-limitative example, wherein:

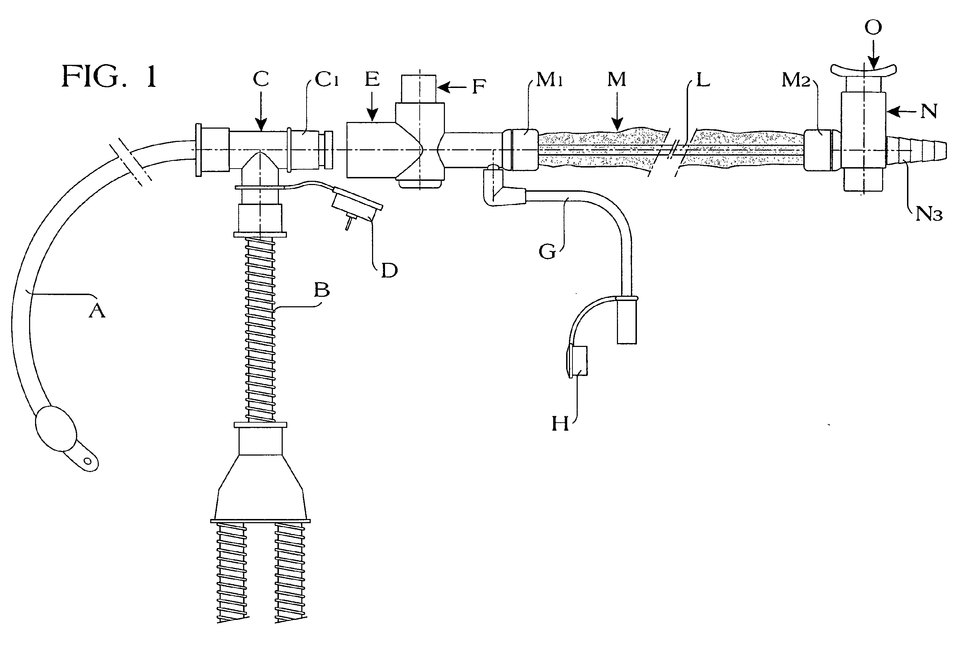

Figure 1 is a view of the closed tracheal suction system before connection to the Cobb connector and therefore to the ventilation circuit, according to the invention;
Figure 2 is a longitudinal sectional view of the Cobb connector;
Figure 3 is a longitudinal sectional view of the connector for coupling the closed tracheal suction system to the Cobb connector;
Figures 4, 5 and 6 are a longitudinal sectional view and two views of the flow control element for the valve for closing the internal lumen of the coupling connector of Figure 3;
Figures 7 and 8 are exploded longitudinal sectional views of the valve to be operated manually in order to connect the tracheal suction system to the suction pump;
Figures 9 and 10 illustrate the closed tracheal suction system, split over two figures due to drawing size requirements, after connection to the Cobb connector and insertion of the tracheal suction catheter in the endotracheal tube;
Figure 11 is a longitudinal sectional view of the opening of the valve that connects the tracheal suction system to the suction pump;
Figures 12 and 13 illustrate the closed tracheal suction system, split over two figures due to drawing size requirements, while performing washing in order to remove the secretions collected in the appropriately provided chamber during the return of the catheter from the endotracheal tube to its protective sleeve.

With reference to the drawings, an endotracheal tube A and a flexible hose B of the ventilation circuit or respiration system are connected to a connection element C, known as Cobb connector, which consists of a T-shaped connector whose end C1, aligned and arranged opposite with respect to the end where the endotracheal tube A is inserted, is shaped and sized so that after removing an appropriately provided closure cap D it can be inserted by pushing in a corresponding frustum-shaped seat E1 of a connecting element E, whose opposite end E2 is instead inserted with a snap action in a rigid collar M1 of a flexible sleeve M that protects a tracheal suction catheter L.

The collar M1 also keeps in abutment an element Q, which is inserted axially inside the end E2 and crossed longitudinally by a tracheal suction catheter K and during the retraction of the catheter from the endotracheal tube A toward the protective sleeve M retains any secretions in the region of the element E that acts as a washing chamber after closing the valve of the element E, which also allows to isolate the catheter from the outside environment when the closed tracheal suction system is separated from the Cobb connector.

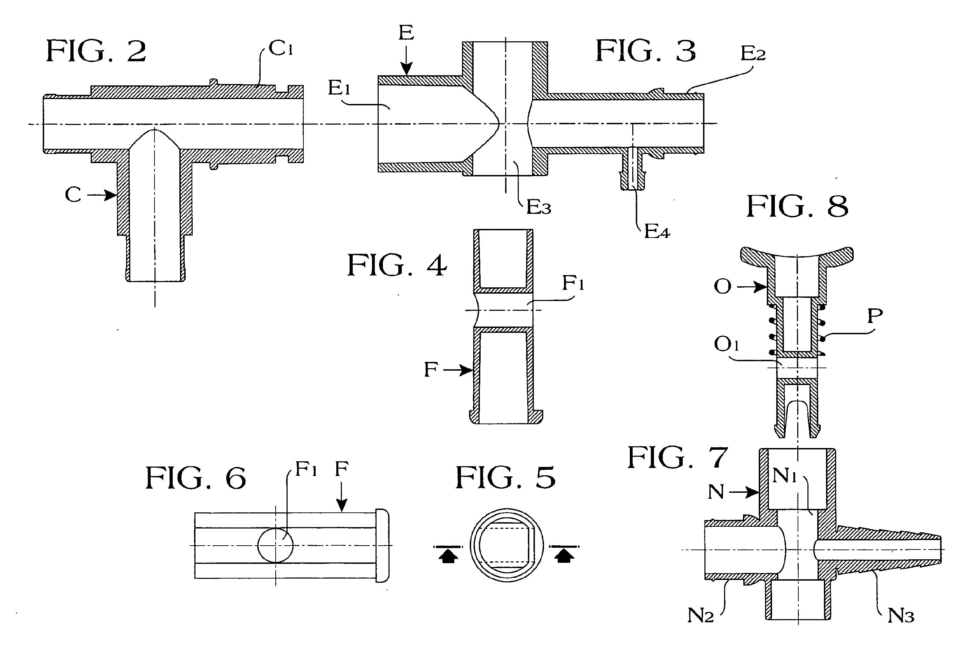

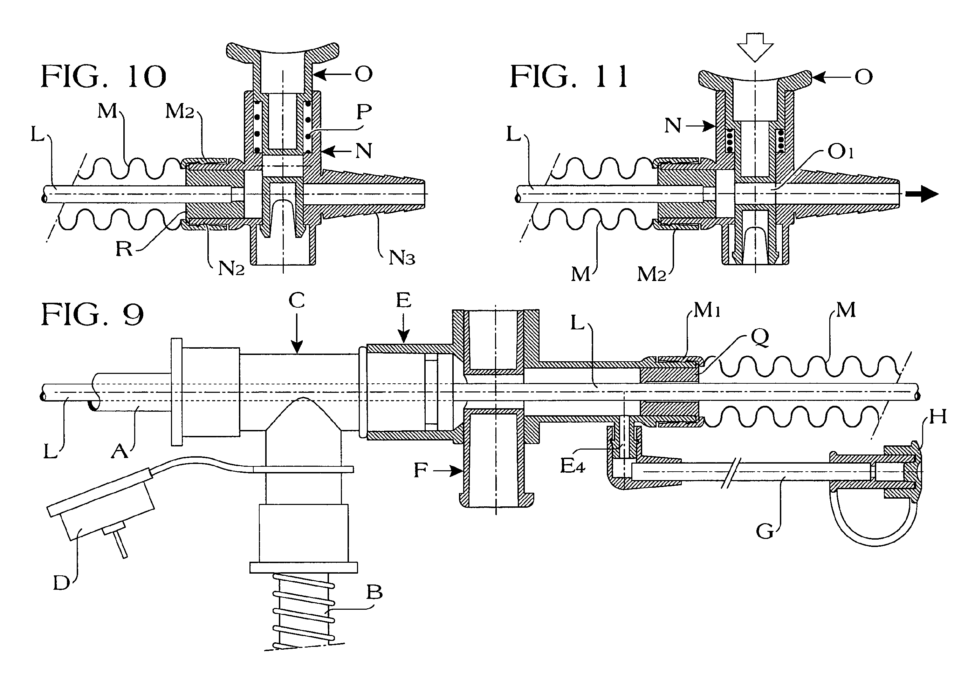

In a seat E3 of the valve (Figure 3), which can be actuated manually, there is the flow control element F (Figures 3, 4, and 5), whose transverse hole F1, when the valve is open as in Figure 9, allows the passage of the tracheal suction catheter L toward the Cobb connector C and the endotracheal tube A.

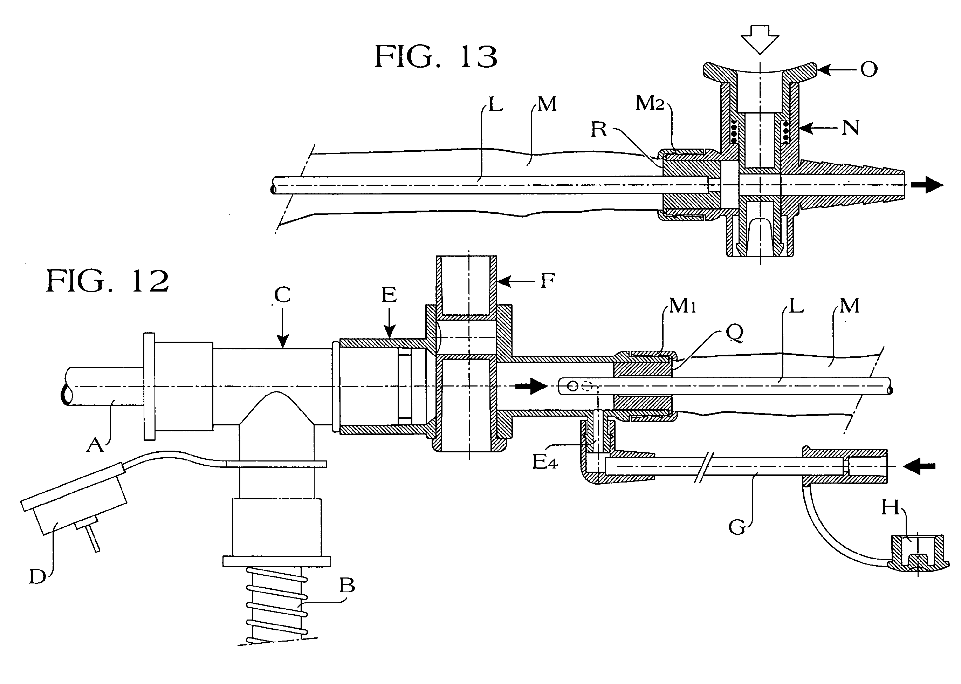

In the washing chamber, i.e., in the region of the connecting element E that lies between the flow control element F of the valve and the catheter scraper element Q, there is a coupling E4 for a tube G provided with a plug H and through which, when necessary, the washing liquid is introduced after extracting the catheter L from the endotracheal tube A and after appropriately placing the flow control element F in the closed position as in Figure 12.

Again with the aim of allowing, when necessary, the perfect isolation of the tracheal suction catheter from the outside environment, the flexible sleeve M, at the end that lies opposite the end connected to the connecting element E, is coupled in a similar manner to the closure valve from a body N of which a coupling N3 protrudes for insertion in the flexible hose for connection to the suction pump.

As shown by Figures 7 and 10, the coupling N2 is in fact inserted with a snap action in a rigid collar M2 of the protective sleeve M of the tracheal suction catheter L, and the collar also keeps an element R, in which the end of the catheter L is fixed firmly, in abutment and inserted in N2.

In the valve for closing the connecting element N, shown in Figures 7, 8 and 10, a flow control element O, which is shaped also to allow its snap coupling in the seat N1 of the body N, is moved manually so as to open, forcing it to perform a translational motion in the seat N1 and in contrast with a helical spring P until its transverse hole O1 is aligned with the couplings N2 and N3 of the body N.

Accordingly, when manual intervention ceases, the spring P automatically returns the flow control element O to the closed position as in Figure 10, i.e., to the limit allowed by its means for engagement in the seat N1.

Clearly, without altering the general characteristics that have been illustrated and described, the improvements according to the invention can be susceptible of modifications and variations, which are in any case within the scope of the appended claims.

The disclosures in Italian Patent Application No. FO2000A000032 from which this application claims priority are incorporated herein by reference.

Where technical features mentioned in any claim are followed by reference signs, those reference signs have been included for the sole purpose of increasing the intelligibility of the claims and accordingly such reference signs do not have any limiting effect on the scope of each element identified by way of example by such reference signs.

## Claims

1. A closed tracheal suction system, comprising a Cobb connector, **characterized in that** said connector consists of a T-shaped connector coupled to an endotracheal tube (A) and to a tube (B) of a ventilation circuit, an end (C1) of said connector, which is the end that is aligned and opposite with respect to a coupling for the endotracheal tube (A), is shaped and sized so that after removing a closure cap (D) it can be inserted by pushing in a corresponding frustum-shaped seat (E1) of a connecting element (E) connected to a flexible protective sleeve (M) of a tracheal suction catheter (L).

2. The system of claim 1, **characterized in that** in the connecting element (E), between the coupling (E1) for the Cobb connector and the coupling (E2) for the protective sleeve (M) of the tracheal suction catheter, there is a closure valve for isolating the catheter from the outside environment when the closed tracheal suction system is separated from the Cobb connector and to prevent, during washing of the catheter and of the collected secretions, both the escape of gas and the passage of water in the endotracheal tube.

3. The system of claim 2, **characterized in that** in the seat (E3) of the valve provided in the connecting element (E), which can be actuated manually, there is the flow control element (F), whose transverse hole (F1), when the valve is open as in Figure 9, allows the passage of the tracheal suction catheter (L) toward the Cobb connector (C) and the endotracheal tube (A).

4. The system of claim 1, **characterized in that** the end (E2) of the connecting element (E), i.e., the end that lies opposite the coupling of the Cobb connector, is inserted with a snap action in the rigid collar (M1) of the flexible sleeve (M) that protects the tracheal suction catheter (L) and said collar also keeps in abutment the element (Q) that is inserted axially in (E2) and crossed longitudinally by the tracheal suction catheter (L) and retains any secretions during the retraction of the catheter from the endotracheal tube (A) toward the protective sleeve (M).

5. The system of the preceding claims, **characterized in that** the element (Q), located inside the end (E2) of the connecting element (E) and crossed longitudinally by the tracheal suction catheter (L), retains any secretions in the washing chamber, i.e., between the flow control element (F) of the valve and the catheter scraper (Q), where the coupling (E4) for the tube (G) is located, the washing liquid being introduced through said tube (G) when necessary after extracting the catheter (L) from the endotracheal tube (A) and after appropriately arranging in the closed position the flow control element (F) as in Figure 12.

6. The system of the preceding claims, **characterized in that** in order to allow perfect isolation of the tracheal suction catheter from the outside environment even after the separation of the suction pump, the flexible sleeve (M), at the end that lies opposite the one connected to the connecting element (E), is connected in a similar manner to a connector (N), with a closure valve, from the body of which the coupling (N3) also protrudes for insertion in the flexible hose for connection to the suction pump.

7. The system of claim 6, **characterized in that** the coupling (N2) of the connecting element (N) is inserted with a snap action in the rigid collar (M2) of the protective sleeve (M) and said collar also keeps in abutment, inserted in (M2), the element (R) in which the end of the catheter (L) is firmly fixed.

8. The system of the preceding claims, **characterized in that** the flow control element (O) of the closure valve of the connecting element (N), which is shaped also so as to be coupled with a snap action in the seat (N1) of the body (N), is moved manually so as to open, forcing it to perform a translational motion in the seat (N1) and in contrast with the helical spring (P) until its transverse hole (O1) aligns with the couplings (N2) and (N3) of the body (N); accordingly, when the manual intervention ceases, the spring (P) returns the flow control element (O) automatically to the closure position, as in Figure 10, or to the limit allowed by its means for engaging the seat (N1).
